(19) Europäisches Patentamt
European Patent Office
Office européen des brevets

(11) Veröffentlichungsnummer: **0 254 990**
**B1**

(12) # EUROPÄISCHE PATENTSCHRIFT

(45) Veröffentlichungstag der Patentschrift: **29.08.90**

(51) Int. Cl.⁵: **C07C 205/20**, C07C 201/06

(21) Anmeldenummer: **87110349.5**

(22) Anmeldetag: **17.07.87**

(54) **Verfahren zur Herstellung von 2.2-Bis-(4-hydroxy-3-nitrophenyl)-hexafluorpropan.**

(30) Priorität: **23.07.86 DE 3624815**

(43) Veröffentlichungstag der Anmeldung:
**03.02.88 Patentblatt 88/5**

(45) Bekanntmachung des Hinweises auf die Patenterteilung:
**29.08.90 Patentblatt 90/35**

(84) Benannte Vertragsstaaten:
**BE CH DE FR GB IT LI NL**

(56) Entgegenhaltungen:
**EP-A- 0 110 420**

**C. Ferri "Reaktionen der organischen Synthese", 1978 GEORG THIEME VERLAG, Stuttgart Seiten 175-177**
**JOURNAL OF POLYMER SCIENCE, POLYMER CHEMISTRY EDITION, Band 20, Nr. 7, 1982 K.S.Y. LAU et al. "Synthesis of polymer intermediates containing the hexafluoropropylidene group via functionalization of 2,2-diphenylhexafloropropane" Seiten 2381-2393**
**CHEMICAL ABSTRACTS, Band 92, Nr. 18, 5. Mai 1980, Columbus, Ohio, USA V. KORSHAK et al. "Synthesis and study of aromatic poly(o-nitro-esters)" Seite 3, Zusammenfassung-Nr. 147 261u**

(73) Patentinhaber: **HOECHST AKTIENGESELLSCHAFT, Postfach 80 03 20, D-6230 Frankfurt am Main 80(DE)**

(72) Erfinder: **Schneider, Klaus-Albert, Dr., Schillerring 30, D-6234 Hattersheim am Main(DE)**
Erfinder: **Siegemund, Günter, Dr., Frankfurter Strasse 21, D-6238 Hofheim am Taunus(DE)**

**EP 0 254 990 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

2.2-Bis-(4-hydroxy-3-nitrophenyl)-hexafluorpropan ist die Verbindung der Formel (I):

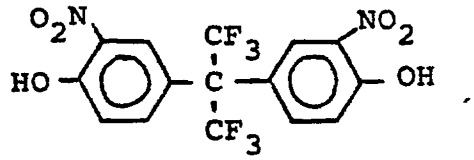

(I)

Sie ist hauptsächlich Zwischenprodukt auf dem Polymerengebiet.

Das Reduktionsprodukt von (I) - die Verbindung 2.2-Bis-(3-amino-4-hydroxyphenyl)-hexafluorpropan (II) - ist u.a. ein Vulkanisationsagenz für bestimmte perfluorierte Elastomere mit noch CN-Gruppen an den Polymermolekülen (EP-A-0110420).

(II) dient auch als Ausgangs-Monomeres für die Polykondensation mit anderen geeigneten polyfunktionellen Verbindungen zur Herstellung chemisch und thermisch außerordentlich stabiler Polymerer (vgl. K.S.Y. Lau et al., Journal of Polymer Science, Polymer Chemistry Edition, Vol. 20, 2381 - 2393 (1982) sowie die dort zitierte Primärliteratur). So werden beispielsweise durch Polykondensation von (II) mit Dicarbonsäuren (wie z.B. mit Terephthalsäure) polymere Benzoxazole erhalten, die sich durch hohe chemische und thermische Stabilität auszeichnen:

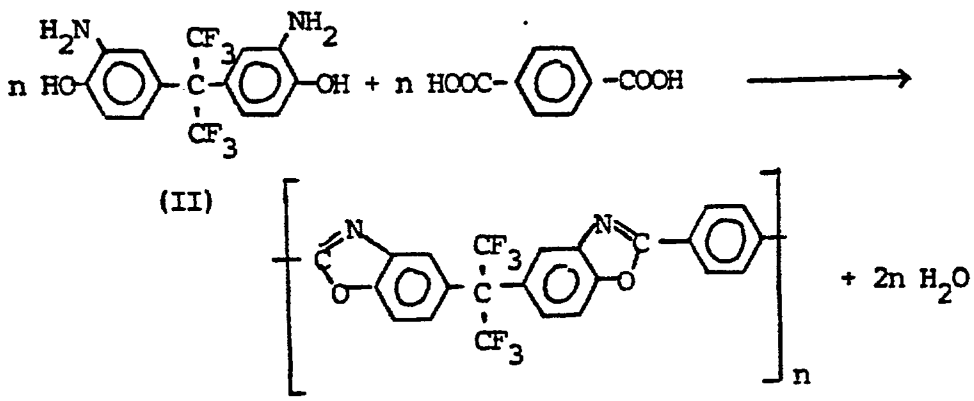

Die Herstellung von (I) erfolgt nach der vorerwähnten EP-A-0110420 durch Nitrierung von 2.2-Bis-(4-hydroxyphenyl)-hexafluorpropan = "Bisphenol AF" mit Kaliumnitrat $KNO_3$/Trifluoressigsäure $CF_3COOH$:

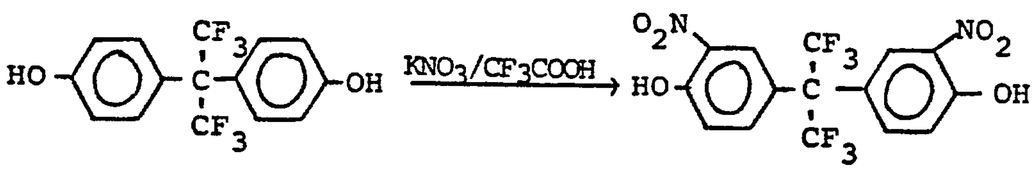


"Bisphenol AF" (I)

Die Rohausbeute an (I) ist nach den beiden diesbezüglichen Beispielen (1 und 2) praktisch quantitativ. Die Angaben in Beispiel 1 lassen auch die Berechnung der Ausbeute an gereinigtem ("bulb-to-bulb distilled") Produkt zu; sie beträgt ca. 77% d.Th.

Trotz der guten Ausbeute ist dieses Verfahren jedoch wegen der Notwendigkeit des Einsatzes der nicht ganz einfach zugänglichen und auch nur mit besonderer Vorsicht handhabbaren Trifluoressigsäure für die Durchführung in technischem Maßstab nur wenig geeignet.

In dem Artikel von K.S.Y. Lau (a.a.O.) ist die Nitrierung mit der auch für den Einsatz in technischen Verfahren besser geeigneten Nitriersäure (= Gemisch aus conc. $HNO_3$ und conc. $H_2SO_4$) beschrieben. Ausgangsverbindung für diese Nitrierung ist jedoch nicht das "Bisphenol AF" als solches, sondern sein Derivat 2.2-Bis-(4-triflatophenyl)hexafluorpropan . Das Derivat muß aus dem "Bisphenol AF" und Trifluormethansulfonsäureanhydrid erst hergestellt werden. Formelmäßig stellt sich dieses Verfahren wie folgt dar:

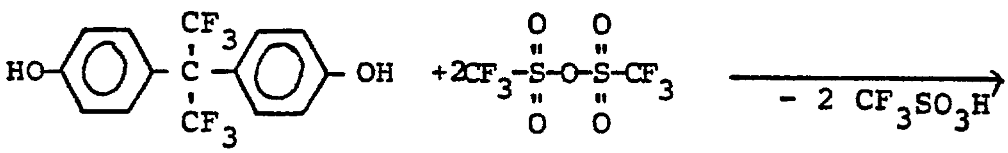


"Bisphenol AF"    Trifluormethan-sulfonsäureanhydrid

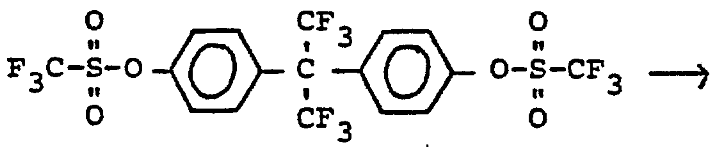

2.2-Bis(triflatophenyl)-hexaflucrpropan

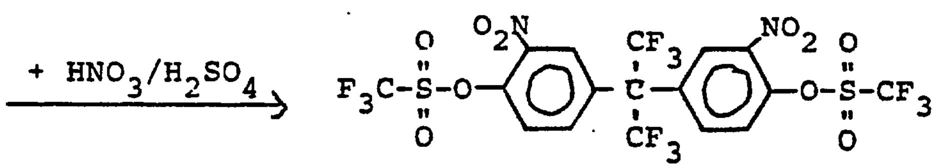


2.2-Bis-(4-triflato-3-nitro-phenyl)-hexafluorpropan

Wie 2.2-Bis-(4-Triflato-3-nitro-phenyl)-hexafluorpropan in (I) überführt wird,ist in der Literaturstelle allerdings nicht beschrieben. Selbst wenn diese Überführung aber beschrieben und auch ohne größeren Aufwand durchzuführen wäre, könnte das Verfahren wegen der Notwendigkeit des nicht leicht zugänglichen und aggresiven Trifluormethansulfonsäureanhydrids kaum mit Vorteil Eingang in die Technik finden.

In dem Bestreben, ein einfacheres und wirtschaftlicheres - auch in technischem Maßstab mit Vorteil durchführbares - Verfahren zur Herstellung von (I) bereitzustellen, wurde nun gefunden, daß sich dieses Ziel durch Nitrierung von "Bisphenol AF" nur mit Salpetersäure - in Abwesenheit anderer Säuren - erreichen läßt.

Erfindungsgegenstand ist daher ein Verfahren zur Herstellung von 2.2-Bis-(4-hydroxy-3-nitrophenyl)-hexafluorpropan (I) durch Nitrierung von 2.2-Bis-(4-hydroxyphenyl)-hexafluorpropan ("Bisphenol AF"),
das dadurch gekennzeichnet ist, daß man die Nitrierung nur mit Salpetersäure - in Abwesenheit anderer Säuren - durchführt.

Formelmäßig läßt sich das Verfahren wie folgt wiedergeben:

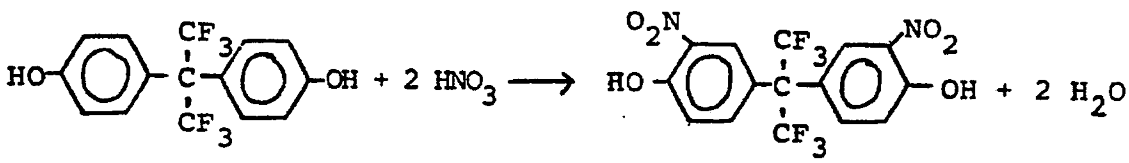

"Bisphenol AF"    (I)

Die nach diesem Verfahren erzielten Ausbeuten an gereinigtem (I) liegen durchweg zwischen etwa 80 und 90%. Das Gelingen dieses sehr einfachen und wirtschaftlichen Verfahrens ist außerordentlich überraschend, weil man nach der vorerwähnten EP-A-0110420 sowie dem Artikel von K.S.Y. Lau et al. (a.a.O.) annehmen mußte, daß man "Bisphenol AF" nicht in einfacher Weise zu (I) nitrieren kann, son-

dern für diese Nitrierung vielmehr kompliziertere Nitriermittel ($KNO_3/CF_3COOH$ -EP-A-0110420) benötigt oder einen nicht ganz einfachen Umweg über ein Derivat des "Bisphenols AF" gehen muß (K.S.Y. Lau et al., a.a.O.).

Die Ausgangsverbindung für das erfindungsgemäße Verfahren - "Bisphenol AF" - kann auf bekannte Weise hergestellt werden (z.B. durch Umsetzung von Hexafluoraceton mit Phenol in Fluorwasserstoff gemäß EP-B-0054227) und ist auch ein im Handel erhältliches Produkt.

Die für das Verfahren eingesetzte Salpetersäure ist vorzugsweise eine 5 bis 98%ige Säure; besonders bevorzugt ist der Einsatz sog. "halbkonzentrierter" - d.i. 50%iger - Salpetersäure.

Die Menge der eingesetzten Salpetersäure wird zweckmäßig so bemessen, daß auf I Mol "Bisphenol AF" 2,5 bis 6 Mol $HNO_3$ kommen; höhere Überschüsse sind im Prinzip möglich, aber kaum mehr von besonderem Vorteil.

Andere Säuren wie z.B. Trifluoressigsäure oder Schwefelsäure, brauchen und sollen nicht - jedenfalls nicht in nennenswerter Menge - mit anwesend sein.

Dagegen ist es ohne weiteres möglich und u.U. sogar von Vorteil, in Gegenwart inerter Lösungs- oder Verdünnungsmittel zu arbeiten; bevorzugt sind hier halogenierte aliphatische Kohlenwasserstoffe wie z.B. $CH_2Cl_2$, $CHCl_3$, $CCl_4$, $ClCH_2$-$CH_2Cl$ insbesondere $CHCl_3$.

Die Temperatur der erfindungsgemäßen Nitrierung wird vorzugsweise zwischen 5 und 30°C, insbesondere zwischen 15 und 25°C gehalten.

Ansonsten wird die Nitrierung in an sich bekannter Weise durchgeführt. Auch die Aufarbeitung erfolgt wie dies für die Aufarbeitung entsprechender Nitrieransätze üblich ist.

Das nachstehende Beispiel soll die Erfindung näher erläutern.

Beispiel

2.2-Bis-(4-hydroxy-3-nitrophenyl)-hexyfluorpropan (I)

In einer Mischung von 302,4 g (0.9 Mol) "Bisphenol AF" und I000 ml Chloroform wurden bei einer Reaktionstemperatur von I5 - 25°C unter kräftigem Rühren 924 g einer aus 439 ml 98%iger Salpetersäure und 56I ml Wasser hergestellten verdünnten Salptersäure getropft. Bei dieser Temperatur wurde zur Vervollständigung der Reaktion eine Stunde nachgerührt. Die organische Phase wurde anschließend abgetrennt, die wäßrige Phase 3 x mit Dichlormethan extrahiert. Die vereinten organischen Phasen wurden mit Natriumhydrogencarbonat-Lösung neutral gewaschen und über $MgSO_4$ getrocknet.

Nach Abziehen des Lösungsmittels wurden 427 g eines zähen Öles als Rohprodukt erhalten - aus welchem nach der Umkristallisation aus Ethanol schließlich 322 g (84% Ausbeute) 2.2-Bis-(4-hydroxy-3-nitrophenyl)-hexafluorpropan (I) isoliert wurden.

Schmp. II6 - II9°C

IR (KBr): $\gamma$ = I540, I340 $cm^{-1}$ ($NO_2$), I280 - II40 $cm^{-1}$($CF_3$)

$^1H$ ($CDCl_3$): $\delta$ = I0.7 (s, breit; 2H, Hydroxyl), 8.3 (d,$^4J_{H,H}$ = 2Hz, 2H, aromt.),
7.6 (dd, $^3J_{H,H}$ = 9 Hz, $^4J_{H,H}$ = 2 Hz, 2H, aromat.), 7.3 (d, $^3J_{H,H}$ = 9 Hz, 2H, aromat.)

$^{13}C$ ($CDCl_3$): $\delta$ = I55.5, I38.I, I33.3, I26.9, I24.3, I20.8 (aromat.), I23.4 (q, $^1J_{C,F}$ = 287 Hz, $CF_3$),
63.3 (sept., $^2J_{C,F}$ = 28 Hz, <u>C</u>($CF_3$)$_2$).

$^{19}F$ ($CDCl_3$): $\delta$ = -64.7 ($CF_3$).

$C_{15}H_8F_6N_2O_6$: Ber.: C 42.3 H 1.9 F 26.7 N 6.6 0 22.5
(426.2) Gef.: C 42.4 H 1.7 F 26.8 N 6.4 0 22.0

**Patentansprüche**

I. Verfahren zur Herstellung von 2.2-Bis-(4-hydroxy-3nitrophenyl)-hexafluorpropan (I) durch Nitrierung von 2.2-Bis-(4-hydroxyphenyl)-hexafluorpropan ("Bisphenol AF"),
dadurch gekennzeichnet, daß man die Nitrierung nur mit Salpetersäure - in Abwesenheit anderer Säuren - durchführt.

2. Verfahren nach Anspruch I, dadurch gekennzeichnet, daß man als Salpetersäure eine 5 bis 95%ige, insbesondere eine 50%ige wäßrige Salpetersäure verwendet.

3. Verfahren nach den Ansprüchen I-2, dadurch gekennzeichnet, daß man die Nitrierung in Gegenwart inerter Lösungsoder Verdünnungsmittel, vorzugsweise von aliphatischen Chlorkohlenwasserstoffen, insbesondere von $CHCl_3$, durchführt.

4. Verfahren nach den Ansprüchen I bis 3, dadurch gekennzeichnet, daß man die Nitrierung bei Temperaturen zwischen 5 und 30°C, vorzugsweise zwischen I5 und 25°C, durchführt.

## Claims

1. A process for preparing 2,2-bis-(4-hydroxy-3-nitrophenyl)-hexafluoropropane (I) by nitrating 2,2-bis-(4-hydroxyphenyl)-hexafluoropropane ("bisphenol AF"), which comprises effecting the nitration with only nitric acid, in the absence of other acids.

2. The process as claimed in claim 1, wherein the nitric acid used is a 5 to 95%, especially a 50% aqueous nitric acid.

3. The process as claimed in claim 1 or 2, wherein the nitration is effected in the presence of inert solvents or diluents, preferably of aliphatic chlorohydrocarbons, especially $CHCl_3$.

4. The process as claimed in any of claims 1 to 3, wherein the nitration is effected at temperatures between 5 and 30°C, preferably between 15 and 25°C.

## Revendications

1. Procédé pour préparer le bis-(hydroxy-4 nitro-3 phényl)-2,2 hexafluoropropane (1) par nitration du bis-(hydroxy-4 phényl)-2,2 hexafluoropropane ("bis-phénol AF"), procédé caractérisé en ce qu'on effectue la nitration au moyen d'acide nitrique seul, sans autres acides.

2. Procédé suivant la revendication 1, caractérisé en ce qu'on utilise, comme acide nitrique, un acide nitrique d'une concentration comprise entre 5 et 95%, plus particulièrement un acide nitrique aqueux à 50%.

3. Procédé suivant l'une des revendications 1 et 2, caractérisé en ce qu'on effectue la nitration en présence de solvants ou diluants inertes, de préférence d'hydrocarbures aliphatiques chlorés, plus particulièrement de $CHCl_3$.

4. Procédé suivant l'une quelconque des revendications 1 à 3, caractérisé en ce qu'on effectue la nitration à des températures comprises entre 5 et 30°C, de préférence entre 15 et 25°C.